# EUROPÄISCHE PATENTANMELDUNG

(11) **EP 1 033 139 A1**
(43) Veröffentlichungstag der Anmeldung: **06.09.2000**
(21) Anmeldenummer: 00102848.9
(22) Anmeldetag: 11.02.2000
(51) Int. Cl.: A61L 9/03

(54) **Vorrichtung zur Abgabe von Duftstoffen**

(30) Priorität: 01.03.1999 DE 29903663 U
(71) Anmelder: Voit, Hans, 82166 Gräfelfing (DE)
(72) Erfinder: Voit, Hans, 82166 Gräfelfing (DE)
(74) Vertreter: Haft, von Puttkamer, Berngruber, Czybulka

(57) **Zusammenfassung**

Eine Vorrichtung zur Abgabe von Duftstoffen weist in einem Gehäuse (1) über einem Vorratsbehälter (19) für eine Duftstofflösung eine mit einer elektrischen Heizeinrichtung (12) erwärmbare, nach oben offene Verdampfungschale (6) und über der Verdampfungsschale (6) einen Ventilator (8) auf, der die Duftstoffe aus dem Gehäuse (1) bläst. Als Dosierpumpe, mit der die Duftstofflösung vom Vorratsbehälter (19) der Verdampfungsschale (6) zugeführt wird, ist eine von einem E-Motor (7) angetriebene Schraubenspindel (25) vorgesehen, die in einem Rohr (24) umläuft, das sich von dem Vorratsbehälter (19) zur Verdampfungschale (6) erstreckt.

## Beschreibung

Die Erfindung bezieht sich auf eine Vorrichtung zur Abgabe von Duftstoffen nach dem Oberbegriff den Anspruchs 1.

Eine derartige Vorrichtung ist bekannt (deutsches Gebrauchsmuster 93 08 368). Die bekannte Vorrichtung weist ein etwa hüfthohes säulenförmiges Gehäuse auf, das am Boden abgestellt wird. Die Dosierpumpe wird durch eine Schlauchpumpe gebildet, die über Schläuche mit dem Vorratsbehälter und der Verdampfungsschale verbunden ist. Eine solche Schlauchpumpe ist auch bei dem deutschen Gebrauchsmuster 298 13 461 vorgesehen, das ein länglich ausgebildetes an einer Wand oder an der Decke befestigbares Gehäuse aufweist.

Die bekannten Duftstoffabgabevorrichtungen haben sich zwar im großen und ganzen bewährt, sie sind jedoch ziemlich voluminös. Zudem sind die Duftstofflösungen zum Teil recht aggressiv, sodass die Schlauchpumpe und die Schläuche mit der Zeit beschädigt werden können.

Aufgabe der Erfindung ist es, eine platzsparende, robuste, einfach aufgebaute Duftstoffabgabevorrichtung bereitzustellen.

Dies wird erfindungsgemäß mit der im Anspruch 1 gekennzeichneten Vorrichtung erreicht. In den Unteransprüchen sind vorteilhafte Ausgestaltungen der erfindungsgemäßen Vorrichtung wiedergegeben.

Bei der erfindungsgemäßen Vorrichtung wird die Dosierpumpe durch eine von einem Elektromotor angetriebene, in einem rohrförmigem Gehäuse umlaufende Förder- oder Schraubenspindel gebildet, wobei sich das Rohr vom Vorratsbehälter in den Boden der Verdampfungsschale erstreckt. Die Förderspindel und das Rohr können damit aus robusten Materialien, wie Metall, oder einem gegenüber der Duftstofflösung beständigen, verschleißfesten Kunststoff bestehen. Auf Schläuche, die mit der Duftstofflösung in Kontakt kommen, kann damit ganz verzichtet werden.

Da zur Förderung der Duftstofflösung mit der Schraubenspindel ein Elektromotor geringer Leistung und damit kleinem Bauvolumen ausreicht, wird der Elektromotor zum Antrieb der Schraubenspindel vorzugsweise zwischen der Verdampfungsschale und dem Ventilator angeordnet.

Der Vorratsbehälter kann damit direkt, jedenfalls mit geringem Abstand unterhalb der Verdampfungsschale angeordnet werden. Damit weist das Gehäuse der erfindungsgemäßen Vorrichtung eine geringe Höhe auf.

Vorzugsweise ist das Gehäuse der erfindungsgemäßen Vorrichtung als Säule ausgebildet. Im Bereich unterhalb der Verdampfungschale weist das Gehäuse vorzugsweise wenigstens eine Lufteintrittsöffnung und auf der gegenüberliegenden Seite oberhalb des Ventilators wenigstens eine Luftaustrittsöffnung auf. Die Oberseite des Gehäuses ist vorzugsweise als von vorne schräg nach oben verlaufende Platte ausgebildet. Die Luftaustrittsöffnung ist dabei vorzugsweise an der Rückseite des Gehäuses angeordnet.

Auf der Platte an der Oberseite des Gehäuses sind vorzugsweise die Bedienungs- und/oder Anzeigeeinrichtung angeordnet. Vorteilhaft sind der Vorratsbehälter mit dem Gewinde, der Deckel mit dem Gegengewinde, die wärmeisolierende Platte, die Verdampfungsschale, das Rohr mit der Schraubenspindel, der E-Motor und/oder der Ventilator koaxial im Gehäuse angeordnet.

Nachstehend ist eine Ausführungsform der erfindungsgemäßen Vorrichtung anhand der Zeichnung beispielhaft näher erläutert. Darin zeigen:
- Fig. 1: eine perspektivische Ansicht der Duftstoffabgabevorrichtung; und
- Fig. 2: eine auseinandergezogene Ansicht der Vorrichtung ohne Gehäuse.

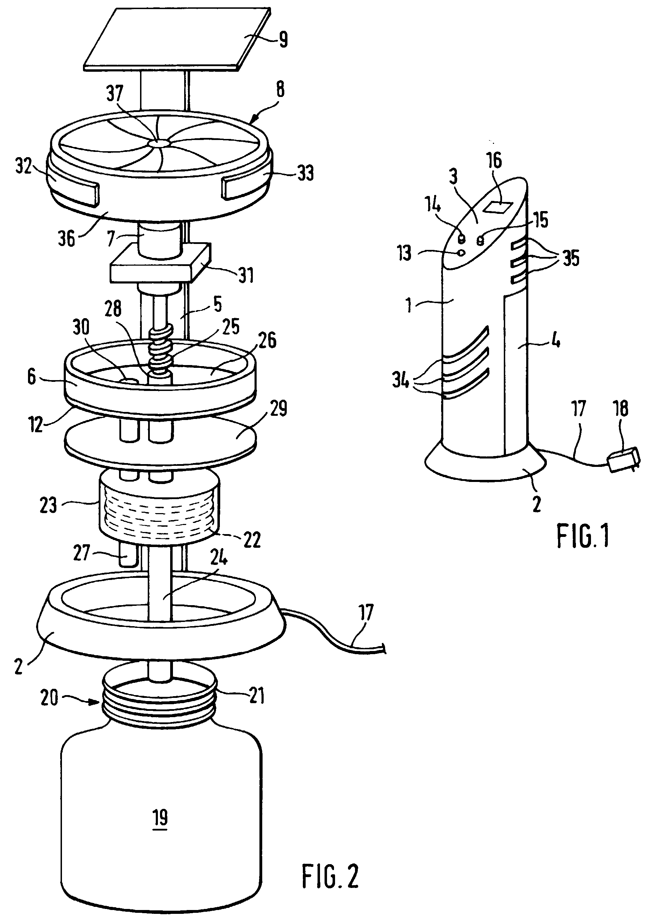

Gemäß Figur 1 weist die Vorrichtung ein säulenförmiges Gehäuse 1 auf, das als Rohr ausgebildet ist. Das Gehäuse 1 ist auf einem Standfuß 2 angeordnet und an seiner Oberseite durch eine schräg von vorne nach oben verlaufende ovale Platte 3 verschlossen. Statt eines kreisförmigen Querschnitts kann das Gehäuse auch rechteckig oder prismenförmig ausgebildet sein.

Die Rückseite des Gehäuses 1 ist mit einer Abdeckung 4 verschließbar, die als schalenförmiges, dem rohrförmigen Gehäuse 1 entsprechendes Segment ausgebildet ist.

Das Gehäuse 1, der Standfuß 2, die Platte 3 und die Abdeckung 4 können aus Metall, z. B. Leichtmetallspritzguß oder Blech hergestellt sein oder aus einem anderen Material bestehen, z. B. Kunststoff. Das Gehäuse 1 mit dem Standfuß 2 weist eine Höhe von beispielsweise 20 bis 50 cm auf.

Gemäß Figur 2 ist der Standfuß 2 als Ring ausgebildet, an dem eine als Schiene ausgebildete Halterung 5 befestigt ist, die sich senkrecht nach oben erstreckt und zwar innen z.B. an der Vorderseite des Gehäuses 1.

An der Schiene 5 sind von unten nach oben eine nach oben offene Verdampfungsschale 6, ein Elektromotor 7, ein Ventilator 8 und eine Platine 9 befestigt.

Die Verdampfungsschale 6 ist durch eine elektrische Heizeinrichtung 12 erwärmbar, die durch eine an der Unterseite der Verdampfungsschale 6 angeordnete Widerstandsheizung, z. B. in Form einer Heizspirale gebildet sein kann.

An der Platine 9 ist die Schaltung zur Steuerung der Vorrichtung angeordnet. Die Platine 9 verläuft schräg, parallel zur Platte 3, die das Gehäuse 1 oben verschließt. An der Platte 3 sind ein Schalter 13, zwei Drehknöpfe 14, 15 und eine Anzeige 16 vorgesehen. Der Schalter 13 dient zum Ein- und Ausschalten der Vorrichtung, sowie zum Einschalten des E-Motors 7 auf Dauerbetrieb bei Reinigung der Vorrichtung. Mit dem Drehknopf 14 wird die Ventilatorgeschwindigkeit eingestellt, und mit dem Drehknopf 15 werden die Zeitintervalle eingestellt, in denen der E-Motor 7 zum Beduften betätigt wird. Beispielsweise kann der Motor 7 zum Beduften alle Stunde einige Sekunden eingeschalten werden. Die Anzeige 16 kann durch eine programmierbare Zeitschaltuhr gebildet sein, die die Zeitintervalle zur Betätigung des Motors 7 vorgibt.

Die Stromzufuhr erfolgt mit einem Kabel 17, mit dem die Vorrichtung an das Stromnetz anschließbar ist. Die elektrischen Einrichtungen, also der E-Motor 7, die Heizeinrichtung 12 und der Ventilator 8 werden durch Schwachstrom, beispielsweise 12 Volt, betrieben. Der dazu erforderliche Transformator kann im Stecker 18 angeordnet sein. Die Stromleitungen können an der Halterung 5 befestigt sein.

Die Duftstofflösung ist in einem Vorratsbehälter 19 vorgesehen, der im Gehäuse 1 unten am Fuß 2 angeordnet wird. Dazu wird der zylinderförmige Vorratsbehälter 19 durch den als Ring ausgebildeten Standfuß 2 von unten nach oben geschoben. Zur Befestigung im Gehäuse 1 weist der Vorratsbehälter 19 an seinem Hals 20 ein Außengewinde 21 auf, das in ein gestrichelt angedeutetes Gegengewinde 22 in einem becherförmigen Deckel 23 angeordnet ist, der entweder direkt oder über die Verdampfungsschale 6 mit der Halterung 5 verbunden ist.

Der durch den ringförmigen Standfuß 2 geschobene Vorratsbehälter 9 kann damit mit seinem Gewinde 21 in das Innengewinde 22 geschraubt und so in dem Gehäuse 1 befestigt werden. Der Vorratsbehälter 19 und der Deckel 23 mit dem Gegengewinde 22 können beispielsweise aus Kunststoff bestehen.

Die Pumpe zum Transport der Duftstofflösung von dem Vorratsbehälter 19 in die Verdampfungsschale 6 erfolgt durch eine durch den E-Motor 7 angetriebene in einem geraden senkrechten Rohr 24 umlaufende Förderschnecke oder Schraubenspindel 25.

Das Rohr 24 erstreckt sich vom unteren Bereich des Vorratsbehälters 19 zum Boden 26 der Verdampfungsschale 6, der dazu mit einer entsprechenden Öffnung versehen ist. Die von der Schraubenspindel 25 geförderte Duftstofflösung aus dem Vorratsbehälter 19 tritt am oberen Endes des Rohres 24 aus und verteilt sich auf dem im wesentlichen ebenen Boden 26 der Schale 6. Die Austrittsöffnung 28 des Rohres 24 ist dazu in der Mitte der Verdampfungsschale 6 angeordnet.

Falls die Duftstofflösung in der Schale 6 nicht vollständig verdampft, fließt sie über ein zum Rohr 24 parallel verlaufendes Rückflußrohr 27 zurück zum Vorratsbehälter 29, das im oberen Bereich des Vorratsbehälters 29 endet. Die Öffnung 30 im Boden 26 der Schale 6, in der das Rückflußrohr 27 endet, ist außermittig angeordnet.

In Figur 2 sind der Deckel 23 und die Schale 6 auseinandergezogen dargestellt. Tatsächlich liegen sie jedoch nahe beieinander, d. h. der Deckel 23 kann auch an der Schale 6 bzw. der Heizung 12 befestigt sein. Um die Schale 3, insbesondere deren elektrische Heizung 12 von dem Deckel 23 bzw. dem Vorratsbehälter 19 thermisch zu trennen, ist eine wärmeisolierende Schicht in Form einer Scheibe 29 aus einem wärmisolierendem Material zwischen der Schale 6 und dem Deckel 23 vorgesehen, wobei die Trennscheibe 29 ebenso wie der Deckel 23 von den Rohren 24 und 27 durchsetzt werden.

Der E-Motor 7 ist in eine kreisförmige Öffnung in einer Halteplatte 31 eingesetzt, die an der Halterung 5 fixiert ist. Die Halterung des Ventilators 8 erfolgt durch das Ventilatorgehäuse 36 am Umfang umgreifende Federarme 32, 33, die an der Halterung 5 befestigt sind.

Die kreisförmige Verdampfungschale 6 kann beispielsweise aus einem Aluminiumwerkstoff bestehen. Der Standfuß 2, der Behälter 19 mit dem Gewinde 21, das Rohr 24 mit der Schraubenspindel 25, der Deckel 23, die Schale 6, die Welle des Motors 7 und/oder die gleichfalls senkrecht angeordnete Welle 37 des Ventilators 8 sind im Gehäuse 1 vorzugsweise koaxial angeordnet.

An der Vorderseite des Gehäuses 1 sind unterhalb des Ventilators 8 im Bereich der Verdampfungsschale 6 und darunter schlitzförmige Lufteintrittsöffnungen 34 vorgesehen, während an der Rückseite 1 des Gehäuses im Bereich oberhalb des Ventilators 8 und unterhalb der schräg verlaufenden Platine 9 schlitzförmige Luftaustrittsöffnungen 35 vorgesehen sind.

Zum Reinigen der Vorrichtung wird der Vorratsbehälter 9 anstelle der Duftstofflösung mit einem Lösungsmittel gefüllt. Mit dem Schalter 13 kann dann der E-Motor 7 auf Dauerbetrieb zum Spülen der Rohre 24 und 27, der Schraubenspindel 25 und der Verdampfungsschale 6 eingeschaltet werden.

Die erfindungsgemäß Vorrichtung kann z. B. auf einem Schrank, in einem Regal, einer Theke oder einem Tisch abgestellt und zum Beduften kleiner Räume, z. B. Büros oder im Privatbereich eingesetzt werden.

## Patentansprüche

1. Vorrichtung zur Abgabe von Duftstoffen, bei der in einem Gehäuse (1) über einem Vorratsbehälter (19) für eine Duftstofflösung eine mit einer elektrischen Heizeinrichtung (12) erwärmbare, nach oben offene Verdampfungschale (6) und über der Verdampfungsschale (6) ein Ventilator (8), der die Duftstoffe aus dem Gehäuse (1) bläst, sowie eine Dosierpumpe vorgesehen sind, mit der die Duftstofflösung vom Vorratsbehälter (19) der Verdampfungsschale (6) zugeführt wird, dadurch gekennzeichnet, dass die Dosierumpe durch eine von einem E-Motor (7) angetriebene, in einem Rohr (24) umlaufende Schraubenspindel (25) gebildet wird, wobei sich das Rohr (24) von dem Vorratsbehälter (19) zur Verdampfungschale (6) erstreckt.

2. Vorrichtung nach Anspruch 1, dadurch gekennzeichnet, dass sich das Rohr (24) in den Boden (26) der Verdampfungsschale (6) erstreckt.

3. Vorrichtung nach Anspruch 1 oder 2, dadurch gekennzeichnet, dass die Austrittsöffnung (28) des Rohres (24) in der Mitte der Verdampfungschale (6) angeordnet ist.

4. Vorrichtung nach einem der vorhergehenden Ansprüche, dadurch gekennzeichnet, dass der E-Motor (7) zum Antrieb der Schraubenspindel (25) oberhalb der Verdampfungsschale (6) und unterhalb des Ventilators (8) angeordnet ist.

5. Vorrichtung nach einem der vorhergehenden Ansprüche, dadurch gekennzeichnet, dass in den Boden (26) der Verdampfungsschale (6) ein Rückflußrohr (27) zum Vorratsbehälter (19) mündet.

6. Vorrichtung nach einem der Ansprüche 1 bis 4 bzw. 5, dadurch gekennzeichnet, dass sich das Rohr (27), in der die Schraubenspindel (25) umläuft, und/oder das Rückflußrohr (27) senkrecht erstrecken.

7. Vorrichtung nach einem der vorhergehenden Ansprüche, dadurch gekennzeichnet, dass das Gehäuse (1) einen ringförmigen Standfuß (2) aufweist, durch den der Vorratsbehälter (19) von unten in das Gehäuse (1) schiebbar ist.

8. Vorrichtung nach Anspruch 7, dadurch gekennzeichnet, dass der Vorratsbehälter (19) ein Schraubgewinde (21) aufweist, das in ein im Gehäuse (1) befestigtes Gegengewinde (22) schraubbar ist.

9. Vorrichtung nach Anspruch 8, dadurch gekennzeichnet, dass das Gegengewinde (22) in einem Deckel (23) vorgesehen ist, der den Vorratsbehälter (19) verschließt.

10. Vorrichtung nach einem der vorhergehenden Ansprüche, dadurch gekennzeichnet, dass zwischen dem Vorratsbehälter (19) bzw. dem Deckel (23) eine wärmeisolierende Schicht (29) angeordnet ist.
